# EUROPEAN PATENT SPECIFICATION

(11) **EP 1 154 795 B1**
(45) Date of publication and mention of the grant of the patent: **17.08.2005**
(21) Application number: 99957453.6
(22) Date of filing: 03.11.1999
(51) Int. Cl.: A61K 45/06, A61K 31/395, A61P 25/14

(54) **MEANS FOR TREATING AND DIAGNOSING RESTLESS LEGS SYNDROME**
VORRICHTUNGEN ZUR BEHANDLUNG UND DIAGNOSE DES RESTLESS LEG SYNDROMS
DISPOSITIF PERMETTANT DE TRAITER ET DE DIAGNOSTIQUER LE SYNDROME DES IMPATIENCES DES MEMBRES INFERIEURS

(30) Priority: 04.11.1998 SE 9803760
(43) Date of publication of application: 21.11.2001
(73) Proprietor: Hedner, Jan, 412 66 Göteborg (SE); Kraiczi, Holger, 411 25 Göteborg (SE)
(72) Inventor: Hedner, Jan, 412 66 Göteborg (SE); Kraiczi, Holger, 411 25 Göteborg (SE)
(74) Representative: Thiel, Christian, Dr. Dipl.-Chem.
(86) International application number: PCT/SE1999/001979
(87) International publication number: WO 2000/025821

(56) References cited:
- WO-A1-98/15267
- WO-A1-98/31362
- DATABASE BIOSIS PREVIEWS, [Online] SILBER MICHEAL H. ET AL.: 'Pergolide in the management of restless legs syndrome: An extended study', XP002946788 Retrieved from dialog information service, File 5, accession no. 11271418 Database accession no. 199800052750 & SLEEP vol. 20, no. 10, October 1997, (ROCHESTER), pages 878 - 882
- DATABASE EMBASE, [Online] DR. M. OECHSNER ET AL.: 'Idiopathic Restless Legs Syndrome: Combination therapy with levodopa and ropinirole', XP002946789 Retrieved from dialog information service, File 73, accession no. 07488355 Database accession no. 1998280298 & AKTUELLE NEUROLOGIE, vol. 25, no. 5, 1998, (GERMANY), pages 190 - 192
- DATABASE EMBASE, [Online] ZOE A. ET AL.: 'High-dose clonidine in a case of restless legs syndrome', XP002946790 Retrieved from diaalog information service, File 73, accession no. 05854145 Database accession no. 1994262109 & ANNALS OF PHARMACOTHERAPY, vol. 28, no. 7/8, 1994, (UNITED STATES), pages 878 - 881
- DATABASE EMBASE, [Online] READ D.J. ET AL.: 'Clonazepam: effective treatment for restless legs syndrome in uraemia', XP002946791 Retrieved from Dialog Information Service, File 73, accession no. 01789295 Database accession no. 1981224248 & BRITISH MEDICAL JOURNAL, vol. 283, no. 6296, 1981, (UNITED KINGDOM), pages 885 - 886

## Description

### FIELD OF THE INVENTION

The present invention relates to a means for treating and diagnosing Restless Legs Syndrome, including Periodic Limb Movements During Sleep, and to a means for carrying out the method.

### BACKGROUND OF THE INVENTION

The Restless Legs Syndrome (RLS) can be described as irresistible leg movements that are often accompanied by creeping sensations deep in the limbs. Occasionally the upper limbs may also be affected. For RLS patients bedtime is particularly associated with the syndrome because rest, particularly lying down in bed, is associated with increased dysesthesia and irresistible leg movements that interfere with sleep onset. Many patients also experience severe paresthesia on awakening in the middle of the night. When symptoms occur, patients move their legs vigorously, flexing, stretching and crossing them one over the other. Often, patients have to get out of bed several times at night and they use various means to relieve themselves of their uncomfortable sensations, such as rubbing, squeezing, or stroking the limb and walking. Several factors are known to modulate the manifestation of RLS including pregnancy, fatigue, very warm environments, prolonged exposure to cold, intake of caffeinated drinks, and smoking. The prevalence of RLS has been estimated to 5% or more of the general population.

A great majority of patients with RLS also experience stereotype repetitive movements once they are asleep, a condition known as Periodic Limb Movements During Sleep (PLMS). However, PLMS also represents a nosological entity distinct from RLS in that many patients with PLMS do not have the other features of RLS. PLMS is best described as rhythmic motor activity during sleep, more frequently occurring in the lower extremities, and less frequently in the upper ones. Each movement typically lasts for approximately 0.5 to 5 seconds with a frequency of about one every 20 to 40 seconds. PLMS clusters last several minutes or even hours. In general, these episodic clusters are more numerous in the first half of the night, but they can recur during the entire sleep period. Intense movements may cause arousal that, if repetitive, can lead to non-restorative sleep and excessive daytime sleepiness. However, mild PLMS can occur without concomitant nocturnal sleep disruption. PLMS is rare in young individuals, but relatively common in the elderly. It was found in 5% of normal subjects between 30 and 50 years of age, 29 per cent of subjects between 50 years and 65 years, and nearly 44 per cent of subjects aged 65 years and older. PLMS is accompanied by a wide variety of sleep-wake complaints including early sleep onset difficulties, nocturnal awakenings and daytime sleepiness. PLMS also occurs in a range of sleep disorders including narcolepsy, central and obstructive sleep apnea syndrome and rapid eye movement (REM) sleep behavior disorder. RLS/PLMS has also been related to several other conditions including myelopathies and peripheral neuropathies associated or not with amyloidosis, diabetes mellitus, anemia, uremia, chronic lung disease, leukemia, rheumatoid arthritis, fibromyositis as well as with several rare neurological conditions. Finally, pharmacological agents such as tricyclic antidepressants and lithium carbonate may induce the disorder as does withdrawal of a variety of drugs such as anticonvulsants, benzodiazepines, barbiturates and other hypnotics.

RLS and PLMS are typically diagnosed by polysomnographic evaluation although clinical history may be helpful for guidance. Standardized tests such as the Suggested Immobilization Test and the Forced Immobilization Test have been used to quantify RLS.

To date, several observations suggest that RLS and PLMS are two clinical manifestations of the same central nervous system dysfunction. However, the exact pathophysiological mechanism remains unknown. The remarkable periodicity of leg movements suggests the presence of an underlying central nervous system pacemaker, possibly at a subcortical location and regulated by rhythmic fluctuations of reticular excitability at an unknown brainstem level. Some publications seem to indicate that signs of EEG arousal often precede EMG-defined motor activity in RIS/PLMS patients. It was suggested that these arousal stimuli trigger EEG alpha activity and leg movements in these patients due to the presence of a lower arousal threshold.

The principal agents described and evaluated for treatment of RLS/PLMS are carbamazepine, the adrenergic agonist clonidine, the γ-aminobutyric acid agonist baclofen, several benzodiazepines including clonazepam, nitrazepam, lorazepam and temazepam, opioid agonists and dopamine agonists. The effect of carbamazepine, clonidine and baclofen were not uniformly documented in all studies but were generally accompanied by a variety of clinically important side effects. Subsequent studies of benzodiazepines have shown a reduction of the number of arousals and awakenings associated with leg jerks, but the number of movements and the PLMS index remained high, suggesting that, whereas sleep may be improved, the actual disease is not affected. Although opiates are potent suppressors of RLS and PLMS the risk for abuse and the danger of addiction limit their clinical use considerably. Both L-dopa and the dopamine receptor agonist bromocriptine were shown to be effective in treating RLS/PLMS when administered shortly before bedtime. The major limitations with these agents relate to efficacy in some patients, since their duration of action is usually to short to effectively cover the whole night. Another potential limitation is the long term adverse effects which may include augmentation of symptoms, arrhytmias, gastrointestinal side effects, psychiatric side effects and hyperkinesia similar to what has been shown in patients with Parkinson's disease treated with L-dopa.

### OBJECTS OF THE INVENTION

As evident from the preceding description of the state of the art, there is a need for an improved method for treating RLS/PLMS. In particular, a new pharmacological treatment of these disorders would offer a definite advantage in front of the methods used at present, many of which provide insufficient relief and some of which are associated with potentially severe side effects and limitations.

One object of the present invention thus is to provide means for the treatment of RLS/PLMS which reduces and/or eliminates some or all of the drawbacks of the methods known in art.

A further object of the present invention is the application of the inventive means as a diagnostic tool for detecting the presence of RLS and/or PLMS in a patient.

Further objects of the invention are evident from the following description of the invention and the appended claims.

### SUMMARY OF THE INVENTION

According to the present invention is provided a medicament for treating and preventing RLS/PLMS, comprising a pharmacologically active amount of an agent having an inhibitory effect on acetylcholine esterase, that is, an acetylcholine esterase inhibitor. For the sake of simplicity, acetylcholine esterase inhibitors will be referred to as choline esterase inhibitors (CEI) in the present application.

For many years CEI have been used in medicine for the treatment of a number of diseases but not for the treatment of RLS/PLMS. The known use of CEI has resulted in the gathering of substantial clinical experience specific to CEI. Known medical indications in which CEI are or have occasionally been used as medicines include intestinal and bladder atonia and myasthenia gravis. In addition, CEI of various structure are widely employed as antidotes to clinically used muscle relaxant agents, in particular curare. For a recent survey in respect of known therapeutic uses of CEI, see: P Taylor, *Anticholinesterase Agents*, in Goodman and Gilman's *The Pharmacological Basis of Therapeutics*, 8th Ed., Pergamon Press, New York etc., 1990. The positive effect of CEI in the treatment of RLS/PLMS may be due to an enhancement of cholinergic transmission in the nervous system which causes the threshold for periodic stimuli impinging in the cerebral cortex to increase, thereby reducing the amount of bursts of alpha activity and leg movements or a modified central nervous processing of afferent/efferent impulses. While this hypothesis provides a scientifically attractive explanation of the observed effect, it should be emphasized that it must not be considered to be binding in any way on the concept and the working of the present invention. Central nervous acetylcholinergic mechanisms are intimately involved in the regulation of wakefulness and sleep. Particularly interesting anatomic structures containing cholinergic nerve cells include the dorsal pontine tegmentum, the thalamus, the cerebral cortex and the hippocampus (see Tononi and Pompelano, Pharmacology of the Cholinergic System. In: *The Pharmacology of Sleep,* Ed. A. Kales, Springer Verlag, Berlin 1995, pp 143-210). In humans, systemic administration of CEI can produce an increase in REM sleep and a shortening of the latency from sleep onset to the first episode of REM sleep. Infusion of the CEI physostigmine was also shown to reduce sedation and induce arousal in the postoperative phase in patients exposed to major surgical intervention. The effect of CEI on central motor control is less well characterized. However, previously published data suggest that natural REM sleep, similar to that seen after cholinergic activation, is associated with a lower frequency of myoclonic jerks in patients with PLMS. An attractive hypothesis for the observed effect of said CEI may therefore be that the central nervous control of motor activity is modified in REM sleep, and that this modification may be reinforced or mimicked by the CEI. An effective amount of a CEI (or a combination of several CEI) is one that eliminates or substantially reduces manifestations of RLS/PLMS over a period of sleep, such as sleep periods of from 10 minutes to 10 hours.

Many agents inhibiting the effect of choline esterase are known in the art. Their chemical structure may vary considerably. CEI particularly useful in the invention include synstigmine, neostigmine, physostigmine, pyridostigmine, ambenon (ambenonium), distigmine, demecarium, edrophonium, tacrine (9-amino-1,2,3,4-tetra-hydroacridine), metrifonate, ecothiopate, eptastigmine, tetrahydrobenzazepine and its alkylcarbamate derivatives, amiridine, linopidine, ENA-713 (rivastigmine, a proprietary drug of Sandoz AG in clinical study for the treatment of Alzheimers disease), velnacrine (a compound in clinical study for the treatment of Alzheimer's disease), Cl-INH (a regulatory glucoprotein having CEI activity), thiabendazole, mitezol, 3,4-diaminopyridine, eseridine, galantamine, including pharmaceutically acceptable salts of those in the aforementioned compounds which are able to form salts with organic or inorganic acids. The aforementioned compounds are fully described in the literature; see, for instance: *Therapeutic Drugs,* C Dollery, Ed., Churchill Livingstone, Edinburgh etc., 1991, and references cited therein. In this publication pharmaceutical compositions useful in the invention are described for a number of CEI. Other CEI useful in the invention include idebenone, besipiridine, and 2,2-dichlorovinyl dimethyl phosphate (DVDP).

Since the choline esterase inhibiting effect of the compounds of the invention, except for non-ionic compounds, usually resides in the nitrogen base part of the agent, the expert in the art will recognize that the desired pharmacological effect will be retained as long as the structure of the nitrogen base remains essentially unchanged. It is thus possible to combine various pharmacologically acceptable acids of said bases to obtain CEI agents having desirable properties from a pharmaceutical formulation standpoint, such as salts being only slightly soluble in aqueous solutions which may be of particular interest in the manufacture of controlled release CEI-preparations.

The CEI mixture of such inhibitors is advantageously formulated in a way appropriate to the chosen administration route. The CEI or mixture of such inhibitors may be administered by various routes. The most preferred route is by peroral administration. In this context the compound of the invention is incorporated in tablets, lozenges, capsules or similar, in particular solid pharmaceutical preparations designed for preferred uptake of the compound through the oral mucosa. Also preferred is absorption within the oral cavity such as sublingual absorption and, consequently, pharmaceutical compositions adapted to such absorption are of particular interest.

Knowledge about clinical pharmacokinetics of CEI (see, for instance: P Hartvig et al., *Clinical Pharmacokinetics of Acetycholinesterase Inhibitors,* in Progress in Brain Research, 84 (1990), 139-14, including secondary references; S-M Aquilonius et al., *Pharmacokinetics and Oral Bioavailability of Pyridostigmine in Man,* Eur. J. Clin. Pharmacol.. 18 (1980) 423-428 is useful in designing CEI preparations for administration to a patient. For this purpose formulation techniques known in the art may be used; in this context reference is made to *Pharmaceutical Dosage Forms: Tablets.* Vol. 1-3, H A Lieberman et al., Eds. Marcel Dekker, New York and Basel, 1989-1990. In particular specific reference is made to chapter 7 (*Special Tablets,* by J W Conine and M J Pikal), chapter 8 (*Chewable Tablets,* by R W Mendes, O A Anaebonam and J B Daruwala), and chapter 9 (*Medicated Lozenges;* by D Peters). Most CEI are salts of quaternary amines or tertiary amines which may form salts with appropriate organic or inorganic acids. In respect of their incorporation as active ingredients in solid or semi-solid pharmaceutical formulations CEI salts can be expected to possess physical properties similar to other kinds of tertiary amine pharmacologically active agents, such as, for example, synthetic anti-muscarinic agents (clinidium salts, hyoscine methobromide, orphenadrine hydrochloride); information in respect of formulation techniques for such anti-muscarinic agents thus is useful in carrying out the present invention (for references, see: Martindale, *The Clinical Pharmacopeia*, 29th Ed., The Pharmaceutical Press, London 1989.

It is also possible to administer the compounds according to the invention by the peroral route in a way in which CEI-absorption will be directed to the gastro-intestinal tract. Appropriate formulations for this variant are also found in the aforementioned publications.

If a choline esterase inhibitor with a short or medium-length pharmacological half-life is used according to this invention, it is desirable to design an oral, buccal or sublingual pharmaceutical formulation for sustained release of the CEI in order to avoid the need for frequent administration which would be particularly difficult during sleep.

A solution for this problem is the fixation, at least for a certain period of time, of a formulation containing the CEI in or near the sublingual region. This can be effected, for instance, by attaching a tablet, lozenge, a slow release formulation or similar to a holding means which is in turn fixed at one or several teeth of the lower jaw. EP 0 230 294 A2 discloses a useful drug applicator intended for application of medicaments in the buccal region; it has the shape of a hollow body externally similar to the crown of one or several teeth. The holder is provided with buccally or lingually disposed orifices and can hold a medicament. Saliva passing into the holder through the orifices dissolves the medicament which is thus slowly dispensed into the appropriate portion of the oral cavity. Another apparatus for trans-mucosal administration is described in US 5,122,127 (Stanley).

It is also possible to incorporate the compound of the invention in polymer matrix, biodegradable or not, from which it could leak slowly into the oral cavity. Appropriate technology for producing biodegradable polyester matrices of the polylactide/polyglycolide type for incorporation and sustained release of pharmacologically active compounds is described in, for instance, L A Sanders et al., J. Pharmaceutical Sci. 75 (1986) 356-360, and in the U.S. Patent No. 3,773,919 (Boswell). Non-degradable polymers of appropriate physical properties can also be used as matrices.

Other devices of absorbing material specifically designed for this purpose such as sponges, non-woven inlays, pieces of woven tissue, felt or other absorbent material useful for slow-release purposes of the compounds according to the invention may be placed under the tongue thus restraining their displacement, or may be fixed to a mandibular dental frame in a buccal or a frontal position. The size and-shape of these absorbing devices should be adapted to decrease the risk of displacement and to minimize discomfort. Such adaptation is also necessary to avoid accidental swallowing or aspiration of the device containing the drug. A device of this sort designed for slow release will extend the potential effect of the drug over periods beyond those limited by the basic pharmacokinetic properties of the agent, thereby maximizing efficiency and duration of treatment which can be extended to cover the entire sleeping period. Where applicable, choline esterase inhibitors may be used in the form of their racemates or as substantially pure enantiomers. Parenteral administration of the CEI according to the invention is also feasible.

The amount of CEI to be administered for treatment of RLS/PLMS will vary depending on factors such as the particular chemical nature of the inhibitor used, the route of administration, the release profile of the formulation into which it is incorporated, the severity of the disease, individual pharmacokinetic and pharmacodynamic properties as well as the status of the patient. For instance, the dose range for peroral administration of donepezil will be in the interval from 0.5 to 30 mg per 24 hours. Normally, an amount of from 2 to 15 mg of donepezil is envisaged as the normal range used for a peroral administration. The appropriate dose range for a particular compound can be determined by titration in routine experiments.

In addition to the methods of administration of the compounds of the invention mentioned above also parenteral, intranasal, and rectal administration is useful, as well as administration by inhalation or transdermal administration The CEI according to the invention can also be effectively administered by inhalation, such as inhalation via the mouth or via the nose but also by, for instance, a nasal spray,. The nasal mucosa is easily accessible by use of extra- or intranasal devices, the later ones appropriately shaped and designed similarly to what has been described above for intraoral and sublingual administration. The transdermal formulation is specifically advantageous in regard of simplicity and from a patient comfort standpoint. In this case, the agent is applied to the skin in form of a viscous ointment or similar. Transdermal administration systems (patches provided with a liquid or semi-liquid pharmaceutical composition) for controlled drug delivery through the skin are well known in the art, for instance for the administration of nicotine and drugs used for diseases of the circulatory system.

The timing of the administration of the composition and/or device comprising the choline esterase inhibiting compound according to the invention will depend on the particular compound, its rate of absorption through the mucosa or the skin, the release profile of the respective sustained release formulation and/or device, if used, and similar. Typically, administration of the CEI will, in the majority of cases, have to start in advance of the sleeping period to achieve optimal effect, for instance 10 minutes to 3 hours prior to the onset of sleep.

The CEI according to the invention may also be combined, in one and the same pharmaceutical preparation, with other pharmacologically active compounds useful in the treatment of RLS/PLMS. The choline esterase inhibitors according to the invention may also be used for diagnosing RLS/PLMS to dissociate them from other types of sleep disorders. The diagnostic means according to the invention comprises a CEI which is to be administered in increasing amounts prior to or during a series of sleep episodes; administration can be in single or multiple doses.
The observation of a reduction of the severity and/or number of RLS/PLMS events or episodes or reduced daytime sleep complaints is indicative of the presence of RLS/PLMS.

Double-blind, placebo controlled parallel group study with donepezil. In a double-blind, placebo controlled parallel study with the CEI donepezil we studied 9 patients with light to moderate RLS/PMLS (PLM index, PLMI = 3-17). Donepezil (5mg once daily, evening dose for 21 days) resulted in a mean reduction of PLMI from 8.3 to 5.3 (approximately 36%). Marked reduction of PLMI was seen in 5 patients (reduction 57-100%), minor change in 3 patients while one patient increased PLMI from the first to the second study night. There was no significant change in total sleep time after treatment while the proportion of rapid eye movement sleep increased with approximately 30% after treatment. No side effects were reported during the study period.

This study demonstrates a potent reduction of PLMI of a CEI agent in patients with RLS/PLMS. Moreover, the study is suggestive of an expected REM sleep promoting effect of CEI.

The study of RLS/PLMS in animal models or in healthy persons may lack reference for patients in which acetylcholine related dysfunction is acquired or genetically determined.

Combination therapy. It is known that CEI have other effects, in particular systemic effects, than those desired in the context of the present invention. These effects are chiefly due to excessive cholinergic stimulation and include increased salivation, nausea and vomiting, abdominal spasms, muscle cramps, bradycardia, increased bronchial secretion, and diarrhea. It is within the scope of the present invention to counteract a specific side effect of this kind by administration of an agent known in the art to be effective in its suppression, for instance an anti-diarrheal agent, such as loperamide hydrochoride, in case of diarrhea, or an anti-vomiting agent, such as domperidone, in the case of nausea and vomiting. Another frequently reported side effect of CEI treatment is insomnia and other sleep disturbances. This effect may be overcome by combining the CEI with one or more out of a number of hypnotic agents including, but not limited to, benzodiazepines, zopiclon, zaleplon and zolpidem. This combination may also offer other advantageous effects in the treatment of RLS/PLMS (see below).

An improved or additive effect of administration of CEI according to the invention can be expected when the medicine is given in combination with an agent known to reduce RLS/PLMS such as carbamazepine, clonidine and baclofen which have been shown to be effective in sporadic reports. Similarly, modifiers of the arousal threshold including, but not limited to, benzodiazepines, zopiclon, zaleplon and zolpidem, and phenothiazines, as well as opioid agents and dopaminergic agents, for instance L-dopa and bromocriptine can also be combined with a CEI. Recognizing that the agents mentioned above do not exert their effects via a primary reaction with the cholinergic system it is within the scope of the present invention to formulate combinations with a CEI in order to treat RLS/PLMS.

For the administration of the CEI in combination with carbamazepine, clonidine, baclofen, a hypnotic agent, an opioid agonist or a dopaminergic agent, a pharmaceutical composition containing both of them can be used or they can be administered in separate pharmaceutical compositions simultaneously or consecutively. Consecutive administration is preferred for pharmacokinetic reasons, such as proper timing of the onset of effect caused by the respective agent. For this purpose sustained or delayed release combinations are preferred.

## Claims

1. Use of an acetyl choline esterase inhibitor (CEI) for the manufacture of a medicament, device, kit or composition for the treatment, prevention or diagnosis of the Restless Legs Syndrome (RLS) or the Periodic Limb Movements During Sleep (PLMS).

2. The use of claim 1, wherein said CEI is selected from synstigmine, neostigmine, physostigmine, pyridostigmine, ambenon (ambenonium), distigmine, idebenone, besipyridine, demecarium, edrophonium, tacrine (9-amino-1,2,3,4-tetrahydroacridine), metrifonate and ecothiopate, eptastigmine, tetrahydrobenzazepine and its alkylcarbamate derivatives, amiridine, linopidine, ENA-713, velnacrine, C1-INH, thiabendazole, mitezol, 3,4-diaminopyridine, eseridine and galantamine, rivastigmine, 2,2-dichlorovinyl dimethyl phosphate (DVDP), including pharmaceutically acceptable salts thereof.

3. The use of claim 2, wherein said CEI is galantamine and/or a pharmaceutically acceptable salt thereof.

4. The use of any of claims 1 to 3, wherein said medicament is adapted for peroral administration of said CEI for gastrointestinal absorption.

5. The use of any of claims 1 to 3, wherein said medicament is adapted for peroal administration of said CEI for sublingual and/or buccal absorption.

6. The use of any of claims 1 to 3, wherein said CEI is adapted for the administration in a form of an ointment or similar for transdermal absorption, preferably in combination with a protective patch.

7. The use of any of claims 1 to 6, wherein said medicament is adapted for administration of said CEI in a dose from 0.1 to 2.500 mg in regard of one major sleeping period, such as a sleeping period of 4 hrs or more.

8. The use of any of claims 1 to 7, wherein said CEI is adapted for the administration in form of a sustained and/or delayed release composition.

9. The use of any of claims 1 to 8, wherein said medicament is adapted for the administration of said CEI in form of a device for controlled release.

10. The use of any of claims 1 to 9, wherein said medicament additionally comprises one or several members of the group consisting of carbamazepine, clonidine, baclofen, hypnotic agent, opioid agonist, and dopaminergic agonist.

11. The use of any of claims 1 to 10, wherein the medicament additionally comprises a therapeutically effective dose of a sleep promoting agent selected from benzodiazepines, zaleplon, zolpidem, zopiclon or other agent interacting with the benzodiazepine receptor complex, phenothiazines, and antihistamines.

## Patentansprüche

1. Verwendung eines Acetylcholinesterase Inhibitors (CEI) zur Herstellung eines Medikamentes, einer Vorrichtung eines Kits oder einer Zusammensetzung zur Behandlung, Prävention oder Diagnose des Restless-Legs-Syndroms (RLS) oder der Periodischen Gliedmaßenbewegung während des Schlafs ("Periodic Limb Movements During Sleep, PLMS").

2. Verwendung nach Anspruch 1, wobei der CEI ausgewählt ist aus Synstigmin, Neostigmin, Physostigmin, Pyridostigmin, Ambenon (Ambenonium), Distigmin, Idebenon, Besipiridin, Demecarium, Edrophonium, Tacrin (9-Amino-1,2,3,4-tetrahydroacridin), Metrifonat und Ecothiopat, Eptastigmin, Tetrahydrobenzazepin und seine Alkylcarbamatderivate, Amiridin, Linopirdin, ENA-713, Velnacrin, C1-INH, Thiabendazol, Mintezol, 3,4-Diaminopyridin, Eseridin und Galantamin, Rivastigmin, 2,2-Dichlorvinyldimethylphosphat (DVDP), einschließlich ihrer pharmazeutisch akzeptablen Salze.

3. Verwendung nach Anspruch 2, wobei der CEI Galantamin und/oder ein pharmazeutisch akzeptables Salz hiervon ist.

4. Verwendung nach einem der Ansprüche 1 bis 3, wobei das Medikament auf die perorale Verabreichung des CEI für die gastrointestinale Absorption abgestimmt ist.

5. Verwendung nach einem der Ansprüche 1 bis 3, wobei das Medikament auf die perorale Verabreichung des CEI für sublinguale und/oder bukkale Absorption abgestimmt ist.

6. Verwendung nach einem der Ansprüche 1 bis 3, wobei der CEI an die Verabreichung in Form einer Salbe oder ähnlichem für die transdermale Absorption abgestimmt ist, vorzugsweise in Verbindung mit einem Schutzpflaster.

7. Verwendung nach einem der Ansprüche 1 bis 6, wobei das Medikament auf die Verabreichung des CEI in einer Dosierung von 0,1 bis 2.500 mg in Bezug auf eine Hauptschlafperiode abgestimmt ist, wie eine Schlafperiode von vier Stunden und mehr.

8. Verwendung nach einem der Ansprüche 1 bis 7, wobei der CEI abgestimmt ist auf die Verabreichung in Form einer Zusammensetzung zur dauerhaften und/oder verzögerten Abgabe.

9. Verwendung nach einem der Ansprüche 1 bis 8, wobei das Medikament abgestimmt ist auf die Verabreichung des CEI in Form einer Vorrichtung für kontrollierte Abgabe.

10. Verwendung nach einem der Ansprüche 1 bis 9, wobei das Medikament zusätzlich ein oder mehrere Mitglieder der Gruppe bestehend aus Carbamazepin, Clonidin, Baclofen, einem Hypnoticum, einem Opioidagonisten und einem dopaminergen Agonisten enthält.

11. Verwendung nach einem der Ansprüche 1 bis 10, wobei das Medikament zusätzlich eine therapeutisch wirksame Dosis eines schlaffördernden Mittels, ausgewählt aus Benzodiazepin, Zaleplon, Zolpidem, Zopiclon oder anderen Verbindungen, die mit dem Benzodiazepin-Rezeptorkomplex interagieren, Phenothiazinen und Antihistaminika, enthält.

## Revendications

1. Utilisation d'un inhibiteur d'acétylcholine estérase (CEI) pour la fabrication d'un médicament, dispositif, kit ou composition en vue du traitement, de la prévention ou du diagnostic du syndrome de l'impatience des membres inférieurs (RLS) ou des mouvements involontaires des membres pendant le sommeil (PLMS).

2. Utilisation selon la revendication 1, dans laquelle le CEI est choisi parmi la synstigmine, la néostigmine, la physostigmine, le pyritostigmine, Ambenon (l'ambénonium), le distigmine, l'idébenone, la besipiridine, le démécarium, l'édrophonium, la tacrine (9-amino-1,2,3,4-tétrahydroacridine), le métrifonate et l'écothiopate, l'eptastigmine, le tétrahydrobenzazépine et ses dérivés alkylcarbamates, l'amiridine, la linopirdine, ENA-713, la velnacrine, C1-INH, le thiabendazole, le mintezol, la 3,4-diaminopyridine, l'éséridine et le galantamine, le rivastigmine, le 2,2-dichlorovinyl diméthyl phosphate (DVDP), y compris leurs sels pharmaceutiquement acceptables.

3. Utilisation selon la revendication 2, dans laquelle le dit CEI est le galantamine et/ou un sel pharmaceutiquement acceptable de celle-ci.

4. Utilisation selon l'une quelconque des revendications 1 à 3, dans laquelle ledit médicament est adapté pour une administration perorale dudit CEI en vue d'une administration gastrointestinale.

5. Utilisation selon l'une quelconque des revendications 1 à 3, dans laquelle ledit médicament est adapté pour une administration perorale dudit CEI en vue d'une absorption sublinguale et/ou buccale.

6. Utilisation selon l'une quelconque des revendications 1 à 3, dans laquelle ledit CEI est adapté pour une administration sous forme de pommade ou similaire pour une absorption transdermique, de préférence en combinaison avec un pansement protecteur.

7. Utilisation selon l'une quelconque des revendications 1 à 6, dans laquelle ledit médicament est adapté pour une administration dudit CEI en une dose de 0,1 à 2500 mg pour une période de sommeil majeure, comme une période de sommeil de 4 heures ou plus.

8. Utilisation selon l'une quelconque des revendications 1 à 7, dans laquelle ledit CEI est adapté pour l'administration sous la forme d'une composition à diffusion continue et/ou retardée.

9. Utilisation selon l'une quelconque des revendications 1 à 8, dans laquelle ledit médicament est adapté pour l'administration dudit CEI sous la forme d'un dispositif de diffusion contrôlée.

10. Utilisation selon l'une quelconque des revendications 1 à 9, dans laquelle ledit médicament comprend de plus un ou plusieurs membres du groupe composé du carbamazépine, du clonidine, du baclofène, d'un agent hypnotique, d'un agoniste opioide et d'un agoniste dopaminergique.

11. Utilisation selon l'une quelconque des revendications 1 à 10, dans laquelle le médicament comprend de plus une dose thérapeutiquement efficace d'un agent promoteur du sommeil choisi parmi les benzodiazépines, le zalepton, le zolpidem, le zopiclone et d'autres agents interagissant avec le complexe récepteur des benzodiazépines, les phénothiazines et les antithistamines.
